# EUROPEAN PATENT SPECIFICATION

(11) **EP 1 339 381 B1**
(45) Date of publication and mention of the grant of the patent: **12.10.2005**
(21) Application number: 01989452.6
(22) Date of filing: 05.11.2001
(51) Int. Cl.: A61K 7/16

(54) **DENTIFRICE COMPOSITION**
ZAHNPUTZZUSAMMENSETZUNG
COMPOSITION DE DENTIFRICE

(30) Priority: 09.11.2000 GB 0027405
(43) Date of publication of application: 03.09.2003
(73) Proprietor: SMITHKLINE BEECHAM PLC, Middlesex TW8 9GS (GB)
(72) Inventor: ALEXANDER, Stephen, Edward GlaxoSmithKline, Weybridge Surrey KT13 0DE (GB); NISBET, Mark, Andrew GlaxoSmithKline, Weybridge Surrey KT13 0DE (GB)
(74) Representative: Reeves, Julie Frances
(86) International application number: PCT/EP2001/012994
(87) International publication number: WO 2002/038119

(56) References cited:
- US-A- 5 670 137
- US-A- 5 855 874
- US-A- 5 882 630

## Description

The present invention relates to a dentifrice composition, in particular to a non-aqueous (anhydrous) dentifrice composition. Such non-aqueous compositions may then suitably contain other materials which are unstable and incompatible with an aqueous environment.

There are many materials which have limited solubility in or even react with the aqueous systems of typical dentifrice formulations. One way of overcoming this problem during formulation is to encapsulate these water sensitive materials to prevent them from interacting with the aqueous component(s) present in the dentifrice formulation. Although encapsulation is a well known and used technique that can be usefully employed in the formulation of dentifrice compositions, it does not completely solve the problem as the encapsulated material frequently contacts water in the remainder of the product due to diffusion or 'capsule fracture'.

Other methods of improving the stability of these materials have been suggested and these include the use of anhydrous compositions.

US 4 988 500 (The Procter & Gamble Company) discloses and claims an anhydrous oral composition comprising a carboxyvinyl polymer, a neutralising agent, a peroxide or perborate compound and an anhydrous humectant. It is however necessary to neutralise the carboxyvinyl polymer in order to obtain dentifrice compositions that provide acceptable viscosity characteristics.

US 4 647 451 (Colgate-Palmolive Company) describes an anhydrous dentifrice containing a polysaccharide gum and a glycerine humectant. Polyethylene glycol is optionally added as a dispersion agent.

US 5 882 630 (SmithKline Beecham) describes an anhydrous dentifrice containing a carboxyvinyl polymer, a humectant, a polyethylene glycol, and an abrasive. Suitable carboxyvinyl polymers described include copolymers of acrylic acid cross-linked with polyallylsucrose, for example Carbopol 974 and 934, Carbopol 974 being preferred.

It has now been discovered that an anhydrous dentifrice which contains a hydroxyethyl cellulose polymer, as a thickening agent, has surprisingly advantageous properties in terms of improved rheology and stability. In particular when formulated with solid excipients (including those incompatible with an aqueous environment) the anhydrous dentifrice has good structure with minimal stringiness. Furthermore the anhydrous dentifrice has relatively high viscosity at low shear rates (required when placed upon a toothbrush) but a lower viscosity at high shear rates (required to allow efficient processing of the dentifrice on a commercial scale). In addition the anhydrous dentifrice exhibits reduced syneresis.

Accordingly, the present invention provides a non-aqueous dentifrice composition comprising a hydroxyethyl cellulose polymer, a humectant, a polyethylene glycol and a dentally acceptable abrasive.

The hydroxyethyl cellulose polymer helps thicken the humectant material and also provides the necessary rheology in order to suspend any solid materials including the abrasive.

The term 'rheology' as used herein is intended to reflect the flow characteristics of the formulation.

Suitable hydroxyethyl cellulose polymers for use in dentifrices of the invention include both high and low viscosity grades with differing levels of ethylene oxide substitution. Suitably the hydroxyethyl cellulose polymer has a particle size range of between 5 and 800 micrometers and preferably between 10 and 250 micrometers. Suitably the hydroxyethyl cellulose polymer has a viscosity (when measured as a 1 % w/w aqueous solution) of between 100 and 6000 mPaS and preferably a viscosity between 100 and 400 mPaS. In a preferred embodiment the hydroxyethyl cellulose polymer is of low viscosity (eg between 100 and 400 mPaS) and fine particle size (eg between 10 and 250 micrometers).

The hydroxyethyl cellulose polymer may be present in the range of from 0.1 to 7.5 % w/w, preferably from 0.3 to 2.0 %, more preferably about 1.0 % by weight of the dentifrice.

Suitable humectants for use in the present invention include glycerine, sorbitol and propylene glycol or mixtures thereof. It is well known that commercially available glycerine may contain between 0.5-2.0% by weight of water which is in association with the glycerine. Typically this amount is between 0.5-1.0% by weight. This small amount of water if bound to the glycerine and is therefore not available to the other ingredients. The skilled person would still consider a composition containing glycerine as being non-aqueous. The humectants should in any case be as anhydrous as possible and preferably used in solid form.

Glycerine is the preferred humectant.

As the humectant is used to make the formulations up to 100 % , the humectant may be present in the range of from 20 to 90% by weight of the dentifrice. Preferably the humectant is present from 35 to 75%, more preferably from 45 to 70 % by weight of the dentifrice.

The polyethylene glycol is selected so that it will reduce any stickiness from the formulation and give a smooth textured product. Suitably, the polyethylene glycol will be selected from PEG 300 and PEG 400. PEG 400 is preferred.

Advantageously, the polyethylene glycol is present in the range of from 0.1 to 40 %, preferably 15 to 20 % by weight of the dentifrice.

It is perhaps more suitable to refer to the ratio of hydroxyethyl cellulose polymer to polyethylene glycol that is required in order to produce a product that is smooth and does not show any signs of stickiness.

Advantageously the ratio of hydroxyethyl cellulose polymer to polyethylene glycol is in the range of 1:5 to 1:20, preferably 1:17.5.

Suitable abrasives for use in the present invention include, for example, silica, zinc orthophosphate, sodium bicarbonate (baking soda), plastic particles, alumina, hydrated alumina, calcium carbonate and calcium pyrophosphate or mixtures thereof.

The silica abrasive may be a natural amorphous silica, for instance diatomaceous earth; or a synthetic amorphous silica such as a precipitated silica, for instance 'Tixosil 53B', manufactured by Rhone Poulenc, or a silica gel, such as a silica xerogel; or mixtures thereof.

Generally, an amount of abrasive suitable for use in the dentifrice composition of the present invention will be empirically determined to provide an acceptable level of cleaning and polishing, in accordance with the techniques well known in the art. Suitably, the abrasive will be present in from about 5 to about 60%, preferably from about 5 to about 30%, by weight of the dentifrice.

Advantageously a thickening agent is present in the formulation to give the product a rheology closer to that of a conventional dentifrice. Suitably the thickening agent is a thickening silica, for instance 'Sident 22S', which is manufactured by Degussa Ltd.

The thickening silica will be in the range of from 0.01 to 10%, preferably 5.0 to 7.0% by weight of the dentifrice.

In a further aspect the dentifrice composition of the present invention further comprises a material that is unstable or incompatible with an aqueous environment.

An example of such a material is a bioactive silica-based glass of the type disclosed in WO 96/10985, WO 97/27158 and WO 99/13852. In an aqueous environment such a bioactive glass releases ions causing a significant increase in pH which can adversely affect the stability (especially upon long term storage) of any excipients contained within the dentifrice. Formulating a bioactive silica-based glass in the anhydrous dentifrice of the present invention prevents the release of ions within the dentifrice thereby controlling the pH and increasing the long-term storage stability of the dentifrice.

Surfactant materials are usually added to dentifrice products to provide cleaning and/or foaming properties. Any conventional surfactant used in dentifrice formulations may be used in the present invention, provided that it can be added as a solid powder, that is not in an aqueous solution.

Suitable surfactants include anionic, cationic, nonionic and amphoteric surfactants.

Suitable nonionic surfactants include, for example polyethoxylated sorbitol esters, in particular polyethoxylated sorbitol monoesters, for instance PEG(40) sorbitan diisostearate, and the products marketed under the trade name 'Tween' by ICI; polycondensates of ethylene oxide and propylene oxide (poloxamers), for instance the products marketed under the trade name 'Pluronic' by BASF-Wyandotte; condensates of propylene glycol; polyethoxylated hydrogenated castor oil, for instance, cremophors; and sorbitan fatty esters.

Suitable anionic surfactants include, for example sodium lauryl sulphate, marketed by Albright and Wilson and known as 'SLS'. This may be obtained and is used in a powder form in the present invention.

A particularly preferred anionic surfactant is sodium methyl cocyl taurate, marketed under the trade name 'Adinol CT 95' manufactured by Croda chemicals.

Advantageously, the surfactant is present in the range 0.005 to 20%, preferably 0.1 to 10%, more preferably 0.1 to 5% by weight of the dentifrice.

Advantageously a dentifrice according to the invention may further comprise an ionic fluorine-containing compound, which may include ionic fluorides, such as alkali metal fluorides, amine fluorides and ionic monofluorophosphates, such as alkali metal monofluorophosphates, and which may be incorporated into the formulation, to provide between 100 and 3000ppm, preferably 500 to 2000ppm of fluoride. Preferably the ionic fluoride or monofluorophosphate is an alkali metal fluoride or monofluorophosphate, for instance sodium fluoride or sodium monofluorophosphate, respectively. Stannous fluoride which is not used in conventional dentifrice formulations owing to its instability in an aqueous environment, may also be used at the above levels.

Calcium glycerophosphate which has been shown to enhance the activity of ionic monofluorophosghates, may be optionally added when the fluoride source is an ionic monofluorophosphate.

It will further be appreciated that if an ionic fluorine-containing compound is incorporated in a dentifrice of the invention, the abrasive should be chosen so that it is compatible with the ionic fluorine-containing compound. Thus, for instance, sodium fluoride is well known in the art to be incompatible with abrasives with comprise excess calcium ions as these cause loss of fluoride as insoluble calcium fluoride. Accordingly an abrasive which is insoluble, for instance, a silica, alumina, zinc orthophosphate or plastic particles, is preferred. Alternatively, a calcium abrasive, for instance calcium carbonate, may be used with an alkali metal monofluorophosphate, sodium monofluorophosphate.

Dentifrices according to the invention may also contain other agents conventionally used in dentifrice formulations, for example colouring agents, whitening agents, for example titanium dioxide; preservatives and sweetening agents. Anti-plaque agents, for example triclosan, chlorhexidine, or cetyl pyridinium chloride, anti-calculus agents, for example pyrophosphate salts, anti-sensitivity agents, for example strontium or potassium salts, polymer enhancing agents, for example Gantrez may also be present if required. Breath freshening agents, for example, sodium bicarbonate and tooth whitening agents, for example hydrogen peroxide and sodium tripolyphosphate may also be included at appropriate levels.

In general, such agents will be in a minor amount or proportion of the formulation, usually present in from 0.001 to 5 % by weight of the composition. Any active ingredient or combination of actives that are unstable or incompatible in any way with aqueous environments may also be added to the formulation of the present invention. Flavouring agents may also be added to the formulations, usually at a typical level of 1.0% by weight of the composition.

Suitable sweetening agents include saccharin, cyclamate and acesulfame K, and may be present in from 0.01 to 0.5%, preferably 0.05 to 0.5% by weight of the dentifrice. An auxiliary sweetener such as a thaumatin may also be included, at a level of from 0.001 to 0.1, preferably 0.005 to 0.05% by weight of the dentifrice. A suitable blend of thaumatins is marketed under the trade name 'TALIN' by Tate and Lyle plc.

Dentifrices according to the invention may also contain an antistain agent. Suitable antistain agents include, for example, carboxylic acids such as those disclosed in US 4 256 731, amino carboxylate compounds such as those disclosed in US 4 080 441, phosphonoacetic acid, as disclosed in US 4 118 474, or polyvinylpyrrolidone as disclosed in WO 93/16681. The antistain agent may be incorporated into the dentifrice formulation or may be provided as a separate composition, for use after the dentifrice.

The dentifrices according to the invention may have an initial viscosity of 25,000 to 200,000 centipoise, (eg 50,000 to 100,000 centipoise) which is essential for producing a product that is comparable to conventional dentifrices that have consumer acceptability. The pH of the formulation when diluted in the ratio of 3:1 with water should suitably be less than 10.0.

The viscosity of the dentifrice is measured using a TF 20 spindle Brookfield Viscometer.

The dentifrices according to the invention may be prepared in a conventional manner by mixing the ingredients thereof in the required proportions and in any order which is convenient and, thereafter and if necessary, adjusting the pH.

The following examples illustrate the invention.

### Example 1 Dentifrice.

| INGREDIENT | % w/w |
|---|---|
| HEC 250 GX | 1.000 |
| THICKENING SILICA | 6.500 |
| ABRASIVE SILICA | 14.000 |
| Na MFP | 0.834 |
| TITANIUM DIOXIDE | 1.000 |
| ADINOL CT 95 | 2.000 |
| SACCHARIN | 0.300 |
| POLYETHYLENE GLYCOL(400) | 17.500 |
| FLAVOUR | 1.000 |
| GLYCERIN | 55.866 |
| TOTAL | 100.000 |

### Example 2 Dentifrice comprising bioactive glass.

| INGREDIENT | %w/w |
|---|---|
| HEC 250 GX | 1.000 |
| TFUCKENING SILICA | 6.500 |
| ABRASIVE SILICA | 14.000 |
| Na MFP | 0.834 |
| TITANIUM DIOXIDE | 1.000 |
| ADINOL CT 95 | 2.000 |
| SACCHARIN | 0.300 |
| POLYETHYLENE GLYCOL(400) | 17.500 |
| FLAVOUR | 1.000 |
| GLYCERIN | 48.366 |
| BIOACTIVE GLASS (<20um) | 7.500 |
| TOTAL | 100.000 |
| Adinol CT 95 is a Tradename of Croda Chemicals. | |

### Example 3 Rheology - Viscometry profiles

Viscometry profiles of dentrifrices can be measured using a controlled stress rheometer with a cone and plate geometry. The following plot is of shear stress versus viscosity. The HEC GXR dentrifrice shows the best profile with high viscosity in the low shear region and lower viscosity in the high shear region. This is followed by HEC HX and then Carbopol 974. The PVP K90 dentrifrice has the worst profle showing very little viscosity difference between the two regions.

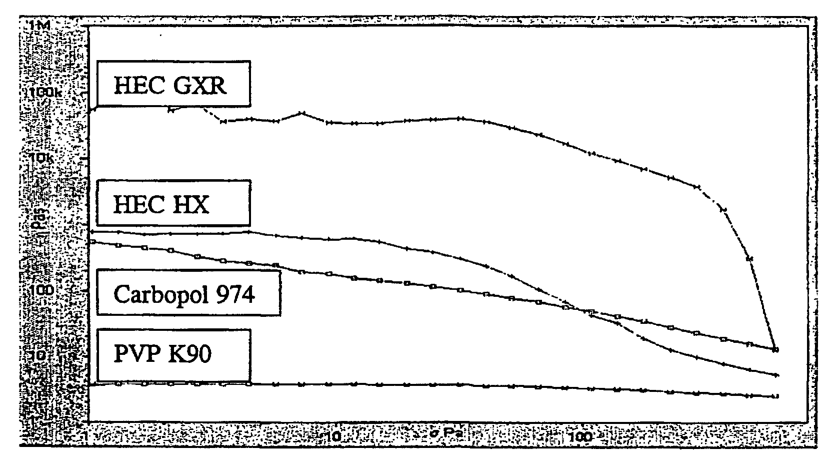

HEC GXR is a low viscosity and fine particle size hydroxyethyl cellulose polymer. HEC HX is a higher viscosity and fine particle size hydroxyethyl cellulose polymer. Carbopol 974 is a carboxyvinyl polymer as disclosed in US-A-5882630.
PVP K90 is a polyvinylpyrrolidone polymer having an approximate molecular weight of 1000000.

## Claims

1. A non-aqueous dentifrice composition comprising a hydroxyethyl cellulose polymer, an anhydrous humectant, a polyethylene glycol and a dentally acceptable abrasive.

2. A composition according to claim 1, wherein the hydroxyethyl cellulose polymer is of a fine particle size.

3. A composition of claim 1 or 2 wherein the hydroxyethyl cellulose has a low viscosity.

4. A composition according to any one of claims 1 to 3, wherein the hydroxyethyl cellulose polymer is present in an amount of from 0.1 to 7.5 % by weight of the dentifrice.

5. A composition according to any one of claims 1 to 4, wherein the anhydrous humectant is glycerine, sorbitol or polyethylene glycol or mixtures thereof.

6. A composition according to claim 5, wherein the anhydrous humectant is present in a range of from 20 to 90% by weight of the dentifrice.

7. A composition according to claim 6, wherein the polyethylene glycol is present in the range of from 0.1 to 40% by weight of the dentifrice.

8. A composition according to any one of the preceding claims, wherein the ratio of hydroxyethyl cellulose polymer to polyethylene glycol is 1:5 to 1:20.

9. A composition according to any one of the preceding claims wherein the abrasive is selected from silica, zinc orthophosphate, sodium bicarbonate, plastic particles, alumina, hydrated alumina, calcium carbonate or calcium pyrophosphate or mixtures thereof.

10. A composition according to any one of the preceding claims, additionally comprising a thickening silica.

11. A composition according to any one of the preceding claims, additionally comprising a material that is unstable or incompatible with an aqueous environment.

12. A composition according to claim 11 comprising a bioactive silica-based glass.

## Patentansprüche

1. Nicht-wäßrige Zahnputzmittelzusammensetzung, die ein Hydroxylethylcellulose-Polymer, ein wasserfreies Befeuchtungsmittel, ein Polyethylenglycol und ein dental annehmbares Schleifmittel umfaßt.

2. Zusammensetzung gemäß Anspruch 1, worin das Hydroxyethylcellulose-Polymer eine feine Teilchengröße hat.

3. Zusammensetzung gemäß Anspruch 1 oder 2, worin die Hydroxyethylcellulose eine niedrige Viskosität hat.

4. Zusammensetzung gemäß einem der Ansprüche 1 bis 3, worin das Hydroxylethylcellulose-Polymer in einer Menge von 0,1 bis 7,5 Gew.% des Zahnputzmittels vorhanden ist.

5. Zusammensetzung gemäß einem der Ansprüche 1 bis 4, worin das wasserfreie Befeuchtungsmittel Glycerin, Sorbit oder Polyethylenglycol oder Mischungen daraus ist.

6. Zusammensetzung gemäß Anspruch 5, worin das wasserfreie Befeuchtungsmittel in einem Bereich von 20 bis 90 Gew.% des Zahnputzmittels vorhanden ist.

7. Zusammensetzung gemäß Anspruch 6, worin das Polyethylenglycol im Bereich von 0,1 bis 40 Gew.% des Zahnputzmittels vorhanden ist.

8. Zusammensetzung gemäß einem der vorhergehenden Ansprüche, worin das Verhältnis von Hydroxylethylcellulose-Polymer zu Polyethylenglycol 1:5 bis 1:20 ist.

9. Zusammensetzung gemäß einem der vorhergehenden Ansprüche, worin das Schleifmittel aus Kieselerde, Zinkorthophosphat, Natriumbicarbonat, Kunststoffteilchen, Aluminiumoxid, Aluminiumhydroxid, Calciumcarbonat oder Calciumpyrophosphat oder Mischungen daraus ausgewählt ist.

10. Zusammensetzung gemäß einem der vorhergehenden Ansprüche, die zusätzlich eine verdickende Kieselerde umfaßt.

11. Zusammensetzung gemäß einem der vorhergehenden Ansprüche, die zusätzlich ein Material umfaßt, das instabil in oder inkompatibel mit einer wäßrigen Umgebung ist.

12. Zusammensetzung gemäß Anspruch 11, die ein bioaktives Glas auf Kieselerdebasis umfaßt.

## Revendications

1. Composition non-aqueuse de dentifrice comprenant un polymère d'hydroxyéthylcellulose, un humectant anhydre, un polyéthylèneglycol et un abrasif acceptable du point de vue dentaire.

2. Composition selon la revendication 1, dans laquelle le polymère d'hydroxyéthylcellulose est de taille particulaire fine.

3. Composition selon la revendication 1 ou la revendication 2, dans laquelle l'hydroxyéthylcellulose a une faible viscosité.

4. Composition selon l'une quelconque des revendications 1 à 3, dans laquelle le polymère d'hydroxyéthylcellulose est présent en quantité variant de 0,1 à 7,5 % en poids par rapport au dentifrice.

5. Composition selon l'une quelconque des revendications 1 à 4, dans laquelle l'humectant anhydre est le glycérol, le sorbitol ou le polyéthylèneglycol ou des mélanges de ceux-ci.

6. Composition selon la revendication 5, dans laquelle l'humectant anhydre est présent dans un intervalle de 20 à 90 % en poids par rapport au dentifrice.

7. Composition selon la revendication 6, dans laquelle le polyéthylèneglycol est présent dans un intervalle de 0,1 à 40 % en poids par rapport au dentifrice.

8. Composition selon l'une quelconque des revendications précédentes, dans laquelle le rapport (polymère d'hydroxyéthylcellulose)/(polyéthylèneglycol) est de 1:5 à 1:20.

9. Composition selon l'une quelconque des revendications précédentes, dans laquelle l'abrasif est choisi parmi la silice, l'orthophosphate de zinc, le bicarbonate de sodium, les particules de plastique, l'alumine, l'alumine hydratée, le carbonate de calcium ou le pyrophosphate de calcium, ou les mélanges de ceux-ci.

10. Composition selon l'une quelconque des revendications précédentes, comprenant en plus, une silice épaississante.

11. Composition selon l'une quelconque des revendications précédentes, comprenant en plus, un matériau qui est instable ou incompatible avec un environnement aqueux.

12. Composition selon la revendication 11, comprenant un verre bio-actif à base de silice.
